# EUROPEAN PATENT SPECIFICATION

(11) **EP 0 748 321 B1**
(45) Date of publication and mention of the grant of the patent: **09.06.1999**
(21) Application number: 95912187.2
(22) Date of filing: 28.02.1995
(51) Int. Cl.: C07D 451/14, A61K 31/435

(54) **PROCESS FOR THE PREPARATION OF AN INDAZOLE-3-CARBOXAMIDE DERIVATIVE**
VERFAHREN ZUR HERSTELLUNG EINES INDAZOL-3-CARBONAMID DERIVATES
PROCEDE DE PREPARATION D'UN DERIVE D'INDAZOLE-3-CARBOXAMIDE

(30) Priority: 03.03.1994 GB 9404055
(43) Date of publication of application: 18.12.1996
(73) Proprietor: SMITHKLINE BEECHAM PLC, Brentford, Middlesex TW8 9EP (GB)
(72) Inventor: LEWIS, Norman, J. SmithKline Beecham Pharma, Tonbridge Kent TN11 9AN (GB); JACEWICZ, Victor, W. SmithKline Beecham Pharma, Tonbridge Kent TN11 9AN (GB); WARD, Neal SmithKline Beecham Pharma, Tonbridge Kent TN11 9AN (GB)
(74) Representative: Waters, David Martin, Dr.
(86) International application number: EP9500764
(87) International publication number: WO9523799

(56) References cited:
- EP-A- 0 081 054
- EP-A- 0 200 444

## Description

The present invention relates to a new process for preparing pharmaceutically active compounds and intermediates therefor.

EP-A-0200 444 (Beecham Group plc) describes certain 5-HT (5-hydroxytryptamine) antagonists which are described as possessing a number of therapeutic utilities, *inter alia* the prevention of vomiting following the administration of cytotoxic agents. The compound described in Example 6 is endo-N-(9-methyl-9-azabicyclo[3.3.1]non-3-yl)-1-methylindazole-3-carboxamide, and this compound has been assigned the INN granisetron. EP-A-0200 444 discloses that granisetron can be prepared by reacting 1-methylindazole-3-carboxylic acid chloride with endo-3-amino-9-methyl-9-azabicyclo[3.3.1]nonane.

A new process has been devised which is particularly selective and can be used to prepare granisetron in a high state of purity.

Accordingly, the present invention provides a process for preparing granisetron (1) or a pharmaceutically acceptable salt thereof: 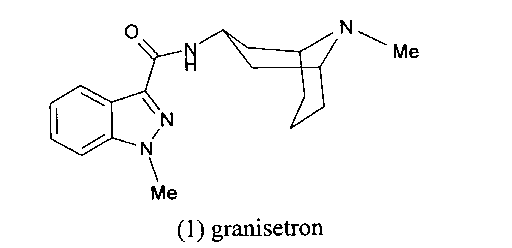 which process comprises deprotecting a compound of Structure (2). 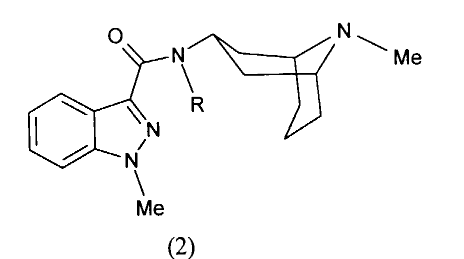 wherein R is benzyl, substituted benzyl, t-butyl, allyl, or t-butyldimethylsilyl, and optionally forming a pharmaceutically acceptable salt.

Substituted benzyl means benzyl substituted with one or more chloro, alkyl or alkoxy groups, preferably methyl or methoxy groups. p-Methoxybenzyl and p-chlorobenzyl are two specific examples.

The reaction is carried out under conditions appropriate to remove the protecting group. For example when R is benzyl or substituted benzyl the reaction may be carried out by treatment under strongly acidic non-aqueous conditions, e.g. methanesulphonic acid, trifluoroacetic acid, or hydrogen chloride or hydrogen bromide in acetic acid. When R is t-butyl the reaction may be carried out under non-aqueous acidic conditions, for example by treatment with trifluoroacetic acid. When R is allyl the reaction may be carried using tetrakis(triphenylphosphine)palladium(0). t-Butyldimethylsilyl can generally be removed by treatment under acidic conditions, for example by treatment with hydrogen chloride in ethanol or trifluoroacetic acid, or by treatment with fluoride ion, for example potassium fluoride or tetrabutylammonium fluoride in a lower alkanol such as methanol.

Compounds of Structure (2) are prepared by reacting an amine of Structure (4), in which R is defined as above, with a compound of Structure (3) in which Q is a leaving group displaceable by a secondary amine. Examples of leaving groups displaceable by a secondary amine include halogen, such as chloro and bromo, C₁₋₆ alkoxy such as methoxy or ethoxy, and N-imidazolyl. Suitably the reaction is carried out at ambient or elevated temperatures in an organic solvent such as toluene or dichloromethane.

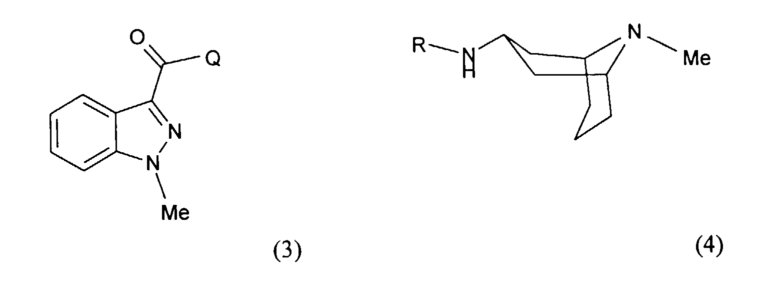

Compounds of Structure (2) are believed to be novel and form part of this invention. Compounds of Structure (4) in which R is benzyl and C₁₋₆alkyl are described in the Journal of the American Chemical Society (1974), volume 96, page 6559 and EP-A-81 054 respectively. Compounds of Structure (4) in which R is benzyl substituted with one or more chloro, alkyl or alkoxy groups, allyl, or a t-butyldimethylsilyl group are believed to be novel and also form part of this invention.

This process is advantageous because of its overall selectivity and in particular it does not give rise to bis-acylated products, compared to the process described in EP-A-0200444.

The compounds of Structure (4) in which R is benzyl or substituted benzyl can be prepared by reducing an imine of Structure (5) (or a tautomeric enamine thereof); suitably the reduction is carried out using sodium borohydride, sodium cyanoborohydride or lithium aluminium hydride, or by selective catalytic hydrogenation, for example using hydrogen and a poisoned palladium catalyst.

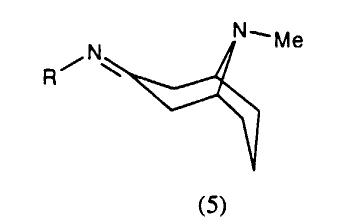

The imines of Structure (5) can be prepared by condensing a ketone of Structure (6) with benzylamine or a substituted benzylamine, or by reacting the amine of Structure (7) with benzaldehyde or a substituted benzaldehyde. Suitably this reaction will be carried out with an acid catalyst under conditions suitable for dehydration e.g. by heating in toluene, or by treatment with titanium tetrachloride or molecular sieve 3A or similar.

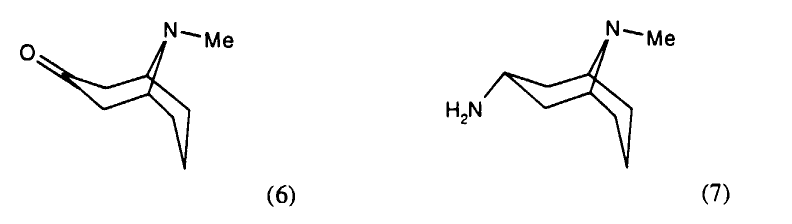

Alternatively the compounds of Structure (4) in which R is benzyl, substituted benzyl or allyl can be prepared by reacting the amine of Structure (7) with a benzyl or allyl halide, for example benzyl chloride or allyl bromide.

In this specification compounds are shown in the boat-chair form. It will be recognised that these conformations will be in equilibrium with the corresponding chair-chair forms which are shown in EP-A-0200444.

The following examples illustrate the present invention.

### Example 1

**a) endo-3-benzylamino-9-methyl-9-azabicyclo-[3,3,1]-nonane** [(4), R = benzyl]
(i) Preparation of intermediate imine: A mixture of endo-3-amino-9-methyl-9-azabicyclo-[3,3,1]-nonane [15.4 g] and benzaldehyde [10.6 g] in toluene [250 ml] was refluxed in a Dean and Stark apparatus for 45 minutes, when the theoretical amount of water was collected. The solvent was removed on a rotary evaporator to give a colourless oil which crystallised on storage in the refrigerator. MS analysis confirmed the molecular weight of the intermediate imine as 242.
(ii) Reduction to the amine: A solution of the intermediate imine [21.0 g] in ethanol [150 ml] was treated with sodium borohydride [3.8 g] in portions over 5 minutes at room temperature, heated briefly to boiling, then cooled. The solvent was removed on a rotary evaporator, the residue taken up in water [250 ml] and the mixture cautiously acidified with concentrated hydrochloric acid [20 ml].

The acidified solution was washed with dichloromethane [250 ml], then made alkaline with sodium hydroxide and extracted twice with dichloromethane [250 ml]. The combined extracts were washed with water [250 ml], dried with magnesium sulphate and evaporated to give the product as a colourless oil [19.0 g].
MS analysis confirmed the molecular weight of the amine as 244, and the NMR spectrum was consistent with the endo-isomer of the title compound.
**b) 1-methylindazole-3-carbonyl chloride** [(3), Q = chloro].
1-Methylindazole-3-carboxylic acid [50.0g] and thionyl chloride [200 ml] were refluxed gently for 2 hours, then allowed to cool. The solution was diluted with hexane [800 ml] to give a pale brown crystalline solid. This was collected, washed on the filter with hexane and dried in the oven at 80°C. Yield 39.3 g (71.2%).
The IR spectrum (Nujol mull) showed a strong band at 1748 cm⁻¹.
**c) N-benzyl-N-(endo-9-methyl-9-azabicyclo-[3,3,1]-non-3-yl)-1-methylindazole-3-carboxamide monohydrochloride** [(2), R = benzyl]
A solution of endo-3-benzylamino-9-methyl-9-azabicyclo-[3,3,1]-nonane [19.0 g] in toluene [150 ml] was added with stirring to 1-methylindazole-3-carbonyl chloride [15.3 g] in toluene [300 ml] at room temperature over 20 minutes. The resulting suspension was stirred at room temperature for 3 hours, the white solid collected by filtration, washed firstly with toluene, then with hexane, and finally dried in the oven at 100°C. Yield 30.70 g (90%).
MS analysis gave the expected molecular weight (402) for the free base of the title compound.
After recrystallisation from isopropanol the product melted at 247 to 248°C and gave the following elemental analysis:

| | | | |
|---|---|---|---|
| Found | C 68.18, | H 7.14, | N 12.89; |
| C₂₅H₃₁N₄ClO requires | C 68.40, | H 7.12, | N 12.76% |

**d) N-(endo-9-methyl-9-azabicyclo-[3,3,1]-non-3-yl)-1-methylindazole-3-carboxamide monohydrochloride [granisetron (1)].**
N-Benzyl-N-(endo-9-methyl-9-azabicyclo-[3,3,1]-non-3-yl)-1-methylindazole-3-carboxamide monohydrochloride (1.00 g) and methane sulphonic acid (5.0 ml) were heated at 70°C with stirring for 1.5 hours. TLC showed a strong spot corresponding to granisetron and confirmed that no starting material remained.
The solution was cooled to room temperature, poured into ice cold water (200 ml), made alkaline using sodium hydroxide and extracted with dichloromethane (100 ml).
The dichloromethane extract was washed twice with water (50 ml), dried over magnesium sulphate and evaporated to a foam. This was treated with toluene (50 ml) and concentrated hydrochloric acid (0.25 g) and again evaporated to dryness. The residue was treated with isopropanol (50 ml) and allowed to crystallise in the cold.
The white solid was collected by filtration, washed with a little isopropanol and dried in the oven at 75°C. Yield 0.40 g (53.8%)
IR and NMR spectroscopy confirmed that the product was granisetron.

### Example 2

**a) endo-3-(4-methoxybenzylamino)-9-methyl-9-azabicyclo-[3,3,1]-nonane** [(4), R = 4-methoxybenzyl]
The reaction of endo-3-amino-9-methyl-9-azabicyclo-[3,3,1]-nonane [15.4 g] and 4-methoxybenzaldehyde [13.6 g] using the conditions described in Example 1(a)(i) gave the intermediate imine as a crystalline solid. MS analysis confirmed the molecular weight as 272.
Reduction of the intermediate imine [27.0 g] in isopropanol [250 ml] with sodium borohydride [3.9 g] following the method of Example I(a)(ii) gave the title compound as a colourless oil [23.0 g]. MS analysis confirmed the molecular weight of the amine as 274, and the NMR spectrum was consistent with the endo-isomer of the title compound.
**b) N-4-methoxybenzyl-N-(endo-9-methyl-9-azabicyclo-[3,3,1]-non-3-yl)-1-methylindazole-3-carboxamide monohydrochloride** [(4), R = 4-methoxybenzyl]
A solution of endo-3-(4-methoxybenzylamino)-9-methyl-9-azabicyclo-[3,3,1]-nonane [14.0 g] in toluene [75 ml] at room temperature was added over 25 minutes with stirring to a solution of 1-methylindazole-3-carbonyl chloride [10.1 g] in toluene [250 ml] at room temperature. The suspension was stirred for 1.5 hours, then allowed to stand overnight The white solid was collected by filtration, washed with toluene, then hexane and dried in the oven at 75°C to give the title compound. Yield 22.1 g (92.3%)
MS analysis gave the expected molecular weight (432) for the free base of the title compound. After recrystallisation from isopropanol the product melted at 233 to 234°C and gave the following elemental analysis:

| | | | |
|---|---|---|---|
| Found | C 65.73, | H 7.03, | N 11.60; |
| C₂₆H₃₃N₄ClO₂. 0.33H₂O requires | C 65.74, | H 7.14, | N 11.80% |

**c) N-(endo-9-methyl-9-azabicyclo-[3,3,1]-non-3-yl)-1-methylindazole-3-carboxamide monohydrochloride [granisetron (1)].**
N-4-Methoxybenzyl-N-(endo-9-methyl-9-azabicyclo-[3,3,1]-non-3-yl)-1-methylindazole-3-carboxamide monohydrochloride (1.00 g) and methane sulphonic acid (5.0 ml) were heated at 70°C with stirring for 20 minutes. TLC showed a strong spot corresponding to granisetron and confirmed that no starting material remained. The cooled mixture was poured into ice cold water, and the product isolated following the method of Example 1(d). Yield 0.57 g (76.6%)
IR and NMR spectroscopy confirmed that the product was granisetron.

### Example 3

### N-(endo-9-Methyl-9-azabicyclo-[3,3,1]-non-3-yl)-1-methylindazole-3-carboxamide monohydrochloride [granisetron (1)].

N-4-Methoxybenzyl-N-(endo-9-methyl-9-azabicyclo-[3,3,1]-non-3-yl)-1-methylindazole-3-carboxamide monohydrochloride (1.00 g) was stirred in trifluoroacetic acid (4.0 ml) at room temperature. After an initial vigorous reaction, the mixture was warmed briefly to 70°C and examined by TLC, which confirmed that the reaction was complete. The mixture was poured into ice cold water (100 ml) and filtered. The filtrate was made alkaline and extracted with dichloromethane (100 ml). The product was isolated from this extract following the method of Example 1(d). Yield 0.41 g (55.0%). IR and NMR spectroscopy confirmed that the product was granisetron.

### Example 4

9-Methyl-9-azabicyclo[3.3.1]nonan-3-one (Ref: Org. Synth Coll Vol. 4, 816) is heated under reflux in toluene with benzylamine with p-toluene sulphonic acid catalysis to give 3-benzimino-9-methyl-9-azabicyclo[3.3.1]nonane. This is reduced with sodium cyanoborohydride in tetrahydrofuran to give 3-benzylamino-9-methyl-9-azabicyclo[3.3.1]nonane. The latter is reacted with 1-methylindazole-3-carboxylic acid chloride in dichloromethane to give N-benzyl-endo-N-(9-methyl-9-azabicyclo[3.3.1]non-3-yl)-1-methylindazole-3-carboxamide.

## Claims

1. A process for preparing granisetron (1) or a pharmaceutically acceptable salt thereof: 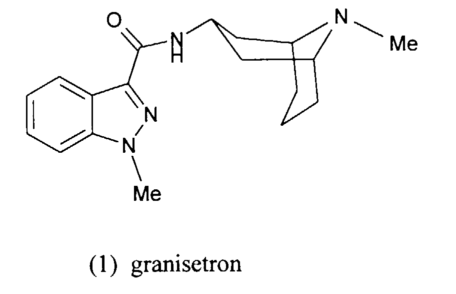 which process comprises the reaction of a compound of formula (3) with a compound of formula (4) 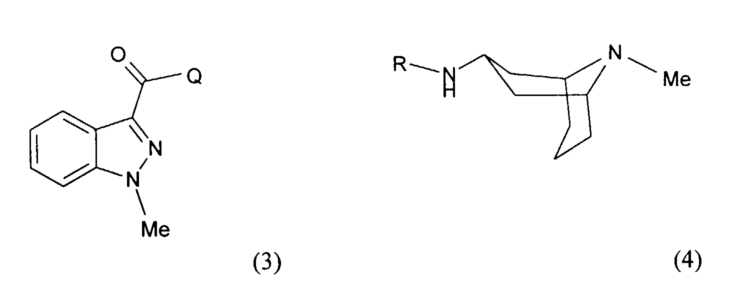 in which Q is a leaving group displaceable by a secondary amine and R is benzyl, benzyl substituted with one or more chloro, alkyl or alkoxy groups, t-butyl, allyl, or a t-butyldimethylsilyl group;
followed by deprotecting the intermediate compound of Structure (2) 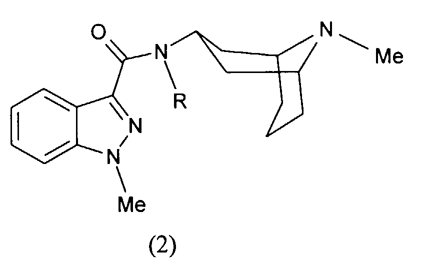 and optionally forming a pharmaceutically acceptable salt.

2. A process according to Claim 1 in which R is benzyl substituted with one or more chloro, methyl or methoxy groups.

3. A compound of Structure (2) 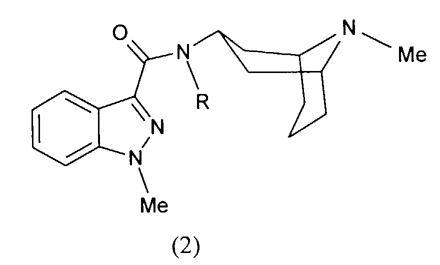 wherein R is benzyl, benzyl substituted with one or more chloro, alkyl or alkoxy groups, t-butyl, allyl, or a t-butyldimethylsilyl group.

4. A compound of Structure (4): 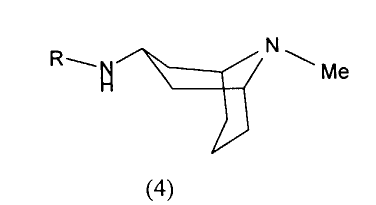 wherein R is benzyl substituted with one or more chloro, alkyl or alkoxy groups, allyl, or a t-butyldimethylsilyl group.

5. A compound according to Claim 3 or Claim 4 in which R is benzyl substituted with one or more chloro, methyl or methoxy groups.

6. A compound of Claim 3 or Claim 4 which is:
(a) N-benzyl-N-(endo-9-methyl-9-azabicyclo-[3,3,1]-non-3-yl)-1-methylindazole-3-carboxamide,
(b) N-4-methoxybenzyl-N-(endo-9-methyl-9-azabicyclo-[3,3,1]-non-3-yl)-1-methylindazole-3-carboxamide, or
(c) endo-3-(4-methoxybenzylamino)-9-methyl-9-azabicyclo-[3,3,1]-nonane, or an acid addition salt thereof.

## Patentansprüche

1. Verfahren zur Herstellung von Granisetron (1) oder eines pharmazeutisch verträglichen Salzes: 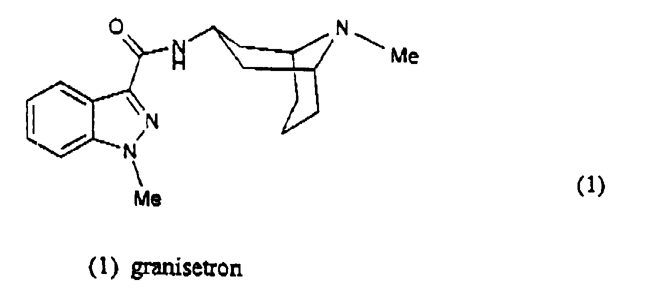 welches Verfahren das Umsetzen einer Verbindung der Formel (3) mit einer Verbindung der Formel (4) 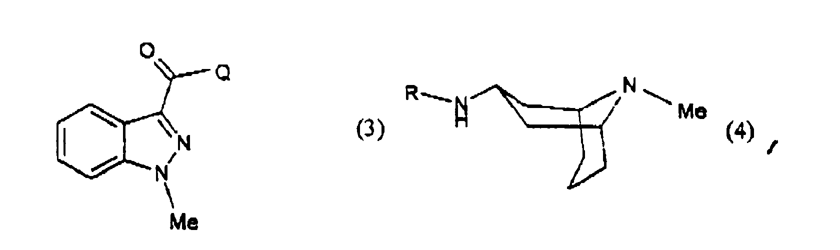 wobei Q eine durch ein sekundäres Amin abspaltbare Abgangsgruppe bedeutet, und R eine Benzylgruppe oder eine mit einem oder mehreren Chloratomen, Alkyl- oder Alkoxyresten substituierten Benzylrest, eine t-Butylgruppe, einen Allylrest oder eine t-Butyldimethylsilylgruppe bedeutet;
danach das Entschützen der Zwischenverbindung der Struktur (2) 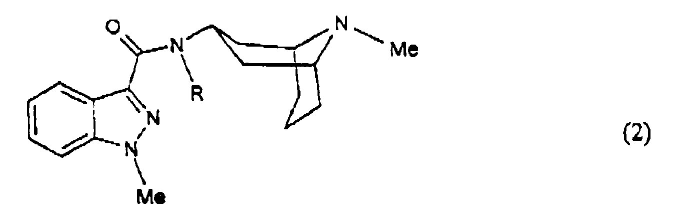 und wahlweise das Ausbilden eines pharmazeutisch verträglichen Salzes umfaßt.

2. Verfahren nach Anspruch 1, wobei R eine mit einem oder mehreren Chloratomen, Methyl- oder Methoxygruppen substituierte Benzylgruppe ist.

3. Verbindung der Struktur (2), 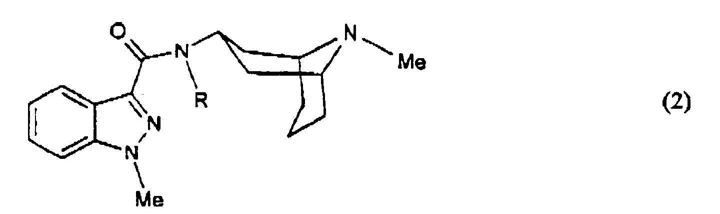 in der R eine Benzylgruppe oder eine mit einem oder mehreren Chloratomen, Alkyl- oder Alkoxyresten substituierten Benzylrest, eine t-Butylgruppe, einen Allylrest oder eine t-Butyldimethylsilylgruppe bedeutet.

4. Verbindung der Struktur (4): 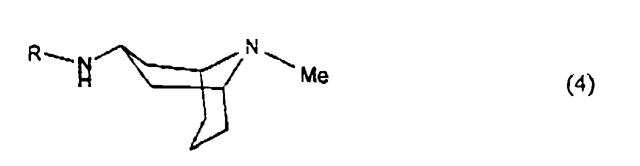 in der R eine mit einem oder mehreren Chloratomen, Alkyl- oder Alkoxyresten substituierter Benzylrest. Allylrest oder eine t-Butyldimethylsilylgruppe ist.

5. Verbindung nach Anspruch 3 oder 4, in der R ein mit einem oder mehreren Chloratomen, Methyl- oder Methoxygruppen substituierte Benzylgruppe ist.

6. Verbindung nach Anspruch 3 oder 4, die
(a) N-benzyl-N-(endo-9-methyl-9-azabicyclo-[3,3,1]-non-3-yl)-1-methylindazol-3-carboxamid,
(b) N-4-methoxybenzyl-N-(endo-9-methyl-9-azabicyclo-[3,3,1]-non-3-yl)-1-methylindazol-3-carboxamid, oder
(c) endo-3-(4-methoxybenzylamino)-9-methyl-9-azabicyclo-[3,3,1]-nonan oder ein Säureadditionssalz ist.

## Revendications

1. Un procédé de préparation de granisetron (1) ou d'un sel pharmaceutiquement acceptable de celui-ci : 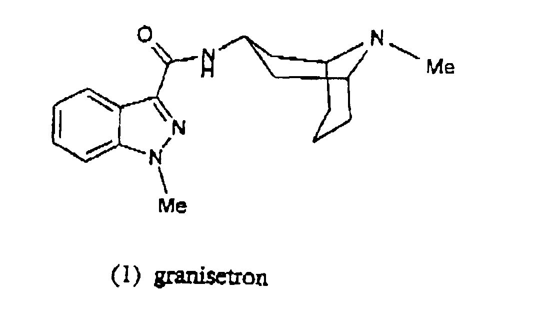 lequel procédé comprend la réaction d'un composé de formule (3) avec un composé de formule (4) 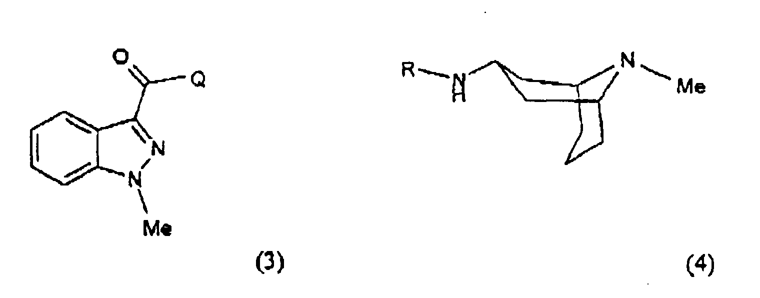 où Q est un groupe partant, susceptible d'être déplacé par une amine secondaire et R représente un groupe benzyle, un groupe benzyle substitué par un ou plusieurs groupes chloro, alkyle, ou alkoxy, un groupe tertio-butyle, allyle ou tertiobutyldiméthylsilyle ; suivie d'une déprotection du composé intermédiaire de structure (2) 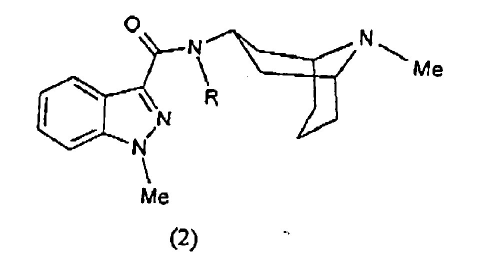 et, facultativement, former un sel pharmaceutiquement acceptable.

2. Un procédé selon la revendication 1, où R est un groupe benzyle substitué par un ou plusieurs groupes chloro, méthyle ou méthoxy.

3. Un composé de structure (2) 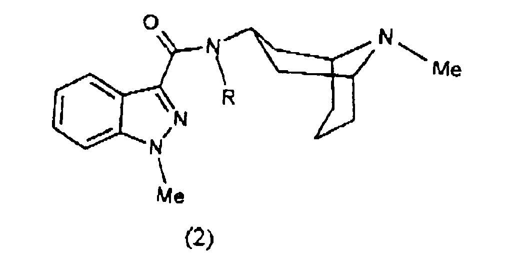 dans lequel R est un groupe benzyle, un groupe benzyle substitué par un ou plusieurs groupes chloro, alkyle ou alkoxy, un groupe tertio-butyle, allyle ou tertio-butyl-diméthylsilyle.

4. Un composé de structure (4) : 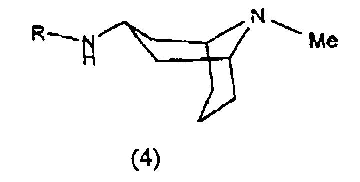 dans lequel R est un groupe benzyle substitué par un ou plusieurs groupes chloro, alkyle ou alkoxy, un groupe allyle ou tertio-butyldiméthylsilyle.

5. Un composé selon la revendication 3 ou 4, dans lequel R est un groupe benzyle substitué par un ou plusieurs groupes chloro, méthyle ou méthoxy.

6. Un composé selon la revendication 3 ou 4, qui est :
(a) du N-benzyl-N-(endo-9-méthyl-9-azabicyclo-[3,3,1]-none-3-yl)-1-méthylindazole-3-carboxamide,
(b) du N-4-méthoxybenzyl-N-(endo-9-méthyl-9-aza-bicyclo-[3,3,1]-none-3-yl)-1-méthylindazole-3-carboxamide, ou
(c) du endo-3-(4-méthoxybenzylamino)-9-méthyl-9-azabicyclo-[3,3,1]-nonane, ou un sel d'addition d'acide de celui-ci.
